# EUROPEAN PATENT APPLICATION

(11) **EP 3 225 997 A1**
(43) Date of publication of application: **04.10.2017**
(21) Application number: 16162545.4
(22) Date of filing: 29.03.2016
(51) Int. Cl.: G01N 33/574

(54) **THSD7A AS A NOVEL TARGET FOR CANCER THERAPY AND DIAGNOSIS**

(71) Applicant: Universitätsklinikum Hamburg-Eppendorf, 20246 Hamburg (DE)
(72) Inventor: STAHL, Rolf, 22359 Hamburg (DE); HOXHA, Elion, 22453 Hamburg (DE); STAHL, Phillip, 20251 Hamburg (DE); WIECH, Thorsten, 20255 Hamburg (DE)
(74) Representative: Uexküll & Stolberg

(57) **Abstract**

The invention relates to a novel marker protein that is expressed by tumor cells. The marker protein Thrombospondin Type-1 Domain-containing 7A (THSD7A) can be used for diagnosing a tumor in a subject by detecting antibodies against this marker protein in the blood of the patient. The invention furthermore relates to an in vitro method of diagnosing cancer which comprises the determination of expression of THSD7A in a subject that is suspected to have cancer and comparison of the level of expression with a standard from a healthy person or a person suffering from cancer. The invention also provides a method of screening drug candidates to identify a drug that is useful for the treatment of cancer which method involves determination of the THSD7A expression level. Finally, the invention provides novel therapeutic approaches for treating THSD7A-expressing cancers.

## Description

The invention relates to a novel marker protein that is expressed by tumor cells. The marker protein Thrombospondin Type-1 Domain-containing 7A (THSD7A) can be used for diagnosing a tumor in a subject by detecting antibodies against this marker protein in the blood of the patient. The invention furthermore relates to an in vitro method of diagnosing cancer which comprises the determination of expression of THSD7A in a subject that is suspected to have cancer and comparison of the level of expression with a standard from a healthy person or a person suffering from cancer. The invention also provides a method of screening drug candidates to identify a drug that is useful for the treatment of cancer which method involves determination of the THSD7A expression level. Finally, the invention provides novel therapeutic approaches for treating THSD7A-expressing cancers.

### FIELD OF THE INVENTION

The small blood vessels in the kidney, i.e. the glomeruli are responsible for filtering waste from the blood. Membranous nephropathy is an autoimmune disease which leads to inflammation in the glomeruli and thickening of their capillary walls. The disease is caused by the deposition of immune complexes on the sub-epithelial side of the glomerular basement membrane. As a result from the inflammation, proteins leak from the blood vessels into the urine, a condition known as proteinuria. The leakage involves the risk of long-term kidney damage, and kidney failure is often the ultimate clinical outcome.

It has been suggested in the literature that there may be an association between membranous nephropathy and malignant tumors (Beck & Salant (2014), J Clin Invest, 124:2307-14). It has been suspected that the immune system massively produces antibodies in response to the tumor which leads to membranous nephropathy. However, a tumor antigen was not identified so that the hypothesis is solely based on the clinical association of the two diseases.

In the course of the present invention, it was found that the protein Thrombospondin Type-1 Domain-containing 7A (THSD7A) in many cases appears to be the target antigen on tumor cells which induces the production of antibodies that leads to membranous nephropathy.

### DESCRIPTION OF THE INVENTION

The insight that THSD7A is expressed on tumors allows the provision of diagnostic and prognostic methods that measure the expression of THSD7A in a tissue of a patient, or the presence of antibodies against THSD7A in a blood sample of a patient.

Therefore, in a first aspect, the invention relates to a method for determining whether a subject is afflicted with cancer or is susceptible to developing cancer, comprising
a) providing a blood sample from a subject; and
b) determining the presence of antibodies against a Thrombospondin type-1 domain-containing 7A (THSD7A) protein in said blood sample of said subject;
wherein the presence of antibodies against a THSD7A protein indicates that said subject is afflicted with cancer or is susceptible to developing cancer.

The method of the invention is conducted with a blood sample that can be obtained from the subject to be tested by commonly known measures, such as venipuncture. Preferably, the subject to be tested is a human subject. However, the method is not limited to humans, but can instead be used for mammalian animals. The blood sample can be a whole-blood sample, or it can be a serum or plasma sample derived from a whole-blood sample. The antibodies to be detected in step (b) of the above method are directed against a THSD7A protein, preferably against a human THSD7A protein. In a particularly preferred embodiment of the invention, the antibodies to be detected are directed against an epitope which is located within the sequence of SEQ ID NO:1. The sequence set out in SEQ ID NO:1 is the amino acid sequence of the human THSD7A protein. The sequence set out in SEQ ID NO:2 is the corresponding genomic sequence that codes for the human THSD7A protein.

Alternatively, the antibodies to be detected in step (b) of the above method may be directed against a THSD7A protein variant which has at least 90% sequence identity to SEQ ID NO:1. As will be appreciated by the person of skill, the THSD7A protein that naturally occurs in humans might differ to some extent between different individuals due to mutations. As a consequence, THSD7A proteins may occur which differ in one or more amino acids from the amino acid sequence in SEQ ID NO:1. These variants of THSD7A are encompassed by the present invention. Hence, as used herein, variants of the protein of SEQ ID NO:1 are proteins with an amino acid sequence that differs from the amino acid sequence of the protein of SEQ ID NO:1 by one or more amino acid substitutions, additions or deletions. In general, any amino acid residue of the sequence shown in SEQ ID NO:1 may be substituted by another amino acid, provided that the resulting protein variant still exhibits an THSD7A activity. Determination of the THSD7A activity can be performed, for example, by measuring the activation of downstream targets of THSD7A. For example, phosphorylation of focal adhesion kinase (FAK) downstream of THSD7A activation can be determined by western blot. In particular, the invention encompasses protein variants which differ from the amino acid sequence shown in SEQ ID NO:1 in up to 5, 10, 15, 20 or even up to 25 amino acid positions.

It is preferred that the substitutions are conservative substitutions, i.e. substitutions of an amino acid residue by another amino acid of a similar polarity, which acts as a functional equivalent. Preferably, the amino acid residue used as a substitute is selected from the same group of amino acids as the amino acid residue to be substituted. For example, a hydrophobic residue can be substituted with another hydrophobic residue, or a polar residue can be substituted with another polar residue having the same charge. Functionally homologous amino acids which may be used for a conservative substitution comprise, for example, non-polar amino acids such as glycine, valine, alanine, isoleucine, leucine, methionine, proline, phenylalanine, and tryptophan. Examples of uncharged polar amino acids comprise serine, threonine, glutamine, asparagine, tyrosine and cysteine. Examples of charged polar (basic) amino acids comprise histidine, arginine and lysine. Examples of charged polar (acidic) amino acids comprise aspartic acid and glutamic acid.

Also comprised by the term "variant" are protein sequences which include more amino acids than the sequence of SEQ ID NO:1, i.e. protein sequences in which one or more amino acids have been inserted. Such insertions may in principle occur at any position of the protein of SEQ ID NO:1. The insertions may be stretches of contiguous amino acids comprising, for example, 2, 3, 4, 5, 6, 7, 8, 9 or ten amino acids. Similarly, the term "variant" also includes protein sequences in which one or more amino acids are deleted as compared to the protein of SEQ ID NO:1. The deletion can involve several contiguous amino acid residues of the protein of SEQ ID NO:1, for example, 2, 3, 4, 5, 6, 7, 8, 9 or ten amino acids. Variants of the protein of SEQ ID NO:1 may also include structural modifications, for example modified amino acids, such as amino acids that have been altered by phosphorylation, glycosylation, acetylation, thiolation, branching and/or cyclization.

The variants of the THSD7A protein set out in SEQ ID NO:1, which have been modified by substitution, addition or deletion of amino acids, normally show a considerable degree of overall amino acid sequence homology or sequence identity to the THSD7A protein of SEQ ID NO:1. Preferably, the homology or identity on the amino acid level between the variant and the THSD7A protein of SEQ ID NO:1 is at least 70%, 80%, 90%, 91%, 92%, 93%, 94%, 95%, 96%, 97%, 98% or 99% if the sequence of the variant is optimally aligned with that of SEQ ID NO:1. Methods and computer programs for determining amino acid homology are well known in the art.

The antibodies may also be directed to a fragment of the THSD7A protein of SEQ ID NO:1 or a fragment of a THSD7A protein variant as described above. This means that the antibodies to be detected are directed to a truncated form of the THSD7A protein.

Different techniques can be used for detecting the antibodies. For example, magnetic beads can be used which comprise recombinantly expressed THSD7A protein on their surface. Antibodies from the blood sample which have bound to the magnetic beads can be visualized by a colorimetric reaction or the like. Other suitable methods for detecting antibodies include immunoblotting, immunofluorescence tests, latex agglutination, enzyme-linked immunosorbent assay, and microparticle enzyme immunosorbent assay. Suitable techniques for qualitatively or quantitatively detecting specific antibodies in blood, plasma or serum have been described in the prior art and moreover belong to the common knowledge of a skilled person.

Where the blood of the subject comprises antibodies against a THSD7A protein, this indicates that said subject is either already afflicted with a cancerous disease or has an increased risk of developing such disease. Thus, in the event that the blood sample contains THSD7A antibodies, the patient should be thoroughly analyzed for the presence of a solid tumor or other forms of cancer, e.g. by scanning the patient's body in a CT scan for tumors. The tumor can be, but is not limited to, a kidney, prostate, urinary bladder, thyroid, esophagus, colon, breast, or lung tumor. In addition, the detection of THSD7A antibodies in the blood of the patient might be indicative for a hematologic cancer, such as leukemia, lymphoma, and multiple myeloma. Where the screening does not result in the identification of a cancerous disease, the patients should be subjected to early detection examination on a regular basis, as these patients have an increased risk of developing a tumor disease.

In a particularly preferred embodiment of the invention, the subject to be tested by the above method is afflicted with membranous nephropathy. In this case, the antibodies produced by the patient's immune system against the THSD7A protein are causative for the membranous nephropathy.

In a second aspect, the invention provides a method of screening drug candidates to identify a drug that is useful for the treatment of cancer, comprising:
a) providing a cell that expresses a THSD7A protein, preferably the sequence of SEQ ID NO:1 or a sequence having at least 90% sequence identity thereto or a fragment of any of these sequences;
b) adding a drug candidate to said cell; and
c) determining the effect of said drug candidate on the expression of said sequence or sequence fragment or on growth, survival, or motility of the cell;
wherein the reduction of expression by the cell or the reduction of growth, survival or motility of the cell indicates that the drug candidate may be useful as a drug for the treatment of cancer.

The screening method of the invention uses in step (a) a cell that expresses a THSD7A protein. The cell to be used in the method of the invention can be any cell that naturally expresses a THSD7A protein, such as a human tumor or non-tumor cell. Alternatively, the cell can be a genetically engineered cell that has been modified by the introduction of an expression vector or another element that provides for the heterologous expression of the THSD7A protein. The engineered cell may be a human or non-human cell, such as a mammalian cell, a bacterial cell, a yeast cell or an insect cell.

In a preferred aspect, the THSD7A protein is expressed in a non-human mammalian cell, e.g. a mouse, rat, hamster, or non-human primate cell. Non-human mammalian cells which have been proven useful for the heterologous expression of polypeptides, preferably human polypeptides, include primary cells and tissues (e.g. murine bone marrow cells, haematopoietic stem cells, murine lymphocytes, muscle tissue of different species, hepatocytes, and the like) as well as cultured cell lines, e.g. Chinese hamster ovary (CHO) cells, monkey kidney cells (COS), baby hamster kidney (BHK) cells, immortalized murine blood lymphocytes and the like.

Mammalian expression systems and appropriate expression constructs are widely known in the art. The expression construct can be, for example, a viral or non-viral expression vector which is useful for introducing the THSD7A-encoding polynucleotides into a cell for subsequent expression of THSD7A. The expression vector can be an episomal vector, i.e. one that is capable of self-replicating autonomously within the host cell, or an integrating vector, i.e. one which stably incorporates into the genome of the cell.

An expression vector will normally comprise a promoter which is functionally linked to the polynucleotide which encodes the THSD7A. Suitable promoters include, but are not limited to, the cytomegalovirus promoter (CMV), the Spleen Focus Forming Virus (SFFV) U3 promoter and the adenoviral major late promoter (Ad MLP). As an optional component, the mammalian expression vector may include a suitable enhancer element for increasing the expression level. Examples include the SV40 early gene enhancer (Dijkema et al. (1985) EMBO J. 4: 761) and the enhancer of the long terminal repeat (LTR) of Rous Sarcoma Virus (Gorman et al. (1982b) Proc. Natl. Acad. Sci. 79: 6777). The expression vector also optionally comprises transcription termination sequences and polyadenylation sequences for improved expression of THSD7A. Suitable transcription terminator and polyadenylation signals can, for example, be derived from SV40 (Sambrook et al (1989), Molecular Cloning: A Laboratory Manual).

In a particularly preferred aspect, the expression vector is a viral expression vector. Viral vectors for use in the present invention typically comprise a viral genome in which a portion of the native sequence has been deleted in order to introduce a heterogeneous polynucleotide without destroying the infectivity of the virus. Due to the specific interaction between virus components and host cell receptors, viral vectors are highly suitable for efficient transfer of genes into target cells. Suitable viral vectors for facilitating gene transfer into a mammalian cell are well known in the art and can be derived from different types of virus, for example, from a retrovirus, adenovirus, adeno-associated virus (AAV), orthomyxovirus, paramyxovirus, papovavirus, picornavirus, or alphavirus.

Alternatively, non-viral vector systems may also be used for introducing the THSD7A-encoding polynucleotide. Non-viral vector systems typically involve non-viral expression vectors which are introduced into the cell, e.g. by liposome-mediated transfer, direct injection of naked DNA, or the gene gun technology.

Several mammalian expression systems are offered by different manufacturers and can be employed in the present invention for expression of THSD7A, such as plasmid- or viral vector based systems, e.g. LENTI-Smart™ (InvivoGen), GenScript^{®} Expression vectors, pAdVAntage™ (Promega), ViraPower™ Lentiviral and Adenoviral Expression Systems (Invitrogen).

The THSD7A protein which is expressed by the cell preferably is a protein of human origin. More preferably, it comprises or consists of the sequence of SEQ ID NO:1. Alternatively, the cell may express a THSD7A protein that is a variant of the sequence of SEQ ID NO:1 as defined elsewhere herein, preferably a variant having a sequence with at least 90% sequence identity to the sequence of SEQ ID NO:1. The cell may also express a fragment of the THSD7A protein of SEQ ID NO:1 or a fragment of one of its variants. Variants and fragments of the THSD7A protein have been defined elsewhere herein.

The screening method of the invention further comprises the addition of a drug candidate to the cell that expresses the THSD7A protein. After contacting the cell with the drug candidate, the effect, if any, of said drug candidate on the cell, in particular the expression of the THSD7A protein or protein fragment is measured. A reduction of expression in cells that were contacted with the drug candidate relative to untreated cells indicates that the drug candidate may be useful as a drug for the treatment of cancer. These drug candidates should be subjected to further examination. Alternatively or in addition, the effect of said drug candidate on growth, survival, or motility of the THSD7A-expressing cell can be determined, and a reduction of growth, survival or motility of the THSD7A-expressing cell indicates that the drug candidate may be useful as a drug for the treatment of cancer. The screening method can be designed as a high-throughput method which allows the testing of several thousand candidate drugs within a short period of time. It is preferred that the reduction of expression is at least 2-fold, preferably at least 5-fold or at least 10-fold.

The drug candidate compounds used in the screening methods of the present invention can include all different types of organic or inorganic molecules, including peptides, oligo- or polysaccharides, fatty acids, steroids, and the like. Typically, the candidate compounds will be small molecules with less than about 2,500 daltons, less than 2000 daltons, less than 1500 daltons, less than 1000 daltons, or less than 500 daltons. Candidate compounds for use in screening methods can be provided in the form of libraries which comprise a high number of synthetic or natural compounds.

In a further aspect, the invention relates to a compound that selectively binds to a THSD7A protein, preferably to the human THSD7A protein of SEQ ID NO:1 or a fragment thereof, for use in a method of treating a THSD7A-expressing cancer disease. The invention for the first time provides the insight that THSD7A is expressed on a number of different tumor cells and can serve as a target for a therapy that aims at the selective killing of these tumor cells. Accordingly, compounds that selectively bind to a THSD7A protein can be used for interfering with the function of THSD7A on the surface of the tumor cells.

The binding compound preferably is an antibody, an antibody fragment, a nanobody or a nanobody fragment. As used herein, an "anti-THSD7A antibody" encompasses any antibody that specifically recognizes the THSD7A protein (or a variant or fragment thereof as defined above) and preferably has the capacity to interfere with the function of the THSD7A protein, such as signal transduction leading to alteration of cell functions or survival. Preferably, THSD7A function is inhibited by the anti-THSD7A antibody by at least 50%, 60%, 70%, 80%, 90%, 95%, 96%, 97%, 98%, 99%, or even more preferably 100%. The antibodies used within the scope of the invention may be any type of antibody, e.g., polyclonal, monoclonal, chimeric, humanized, synthetic or anti-idiotypic antibodies. Suitable anti-THSD7A antibodies can belong to any of the five different classes of antibodies, i.e. IgG, IgA, IgM, IgD and IgE. Preferably, the THSD7A antibodies for use in the therapeutic methods of the invention are derived from the class IgG, more preferably from the sub-classes IgG1, IgG2, IgG2a, IgG2b or IgG3. The antibodies can be obtained from any mammal that has been described in the art in connection with the production of antibodies, e.g., rabbits, rats, mice, goats, horses, primates or humans. Alternatively, the antibodies may be synthetic antibodies which can be produced, for example, by recombinant processes.

According to a preferred aspect, the anti-THSD7A antibody is a monoclonal antibody. As used herein, a "monoclonal antibody" refers to an immunoglobulin which is produced by a cell line which is based on a single B lymphocyte. A monoclonal anti-THSD7A antibody is directed against a single epitope in a THSD7A-antigen. The production of monoclonal antibodies based on hybridoma cell lines has been extensively described in the art (Köhler and Milstein, Nature, 256, 495-397, (1975); Harlow and Lane "Antibodies": Laboratory Manual, Cold Spring Harbor Laboratories (1988); Ausubel et al., (ed.), 1998, Current Protocols in Molecular Biology, John Wiley & Sons, New York). Apart from methods based on hydridoma cells, other methods for the production of monoclonal antibodies have been described. These methods are equally suitable for the production of antibodies for use in the context of the invention. For example, monoclonal antibodies can be produced by recombinant DNA processes as described, e.g. in US Patent 4,816,567. Monoclonal antibodies may also be isolated from an antibody phage library such as described by Clackson et al. (1991), Nature, 352:624-628. Based on the comprehensive literature available, the skilled person will have no problems to prepare an anti-THSD7A antibody for therapeutic use.

When the subject to be treated with the antibody is a human, the anti-THSD7A antibody to be used may be humanized. Humanized antibodies comprise only a minimal sequence which is of non-human origin. The major part of the sequence of a humanized antibody is derived from a human immunoglobulin sequence. Normally, humanized antibodies are produced based on human immunoglobulins in which one or more amino acids from the complementarity-determining region (CDR) are replaced by corresponding amino acids of a non-human antibody having the desired binding characteristics. Methods for producing humanized antibodies have been described in the prior art and are known to the skilled person (see, e.g. Jones et al. (1986) Nature 321: 522-525; Verhoeyen et al. (1988) Science 239: 1534-1536). The humanized antibodies contemplated for use in the therapeutic method of the invention can also comprise additional amino acids which are not present in the recipient antibody or in the donor antibody. These modifications may become desirable to modify (i.e. increase or decrease) the affinity of the humanized antibody. In a preferred embodiment, the humanized antibody comprises hypervariable regions from a non-human immunoglobulin, while all or substantially all of the framework regions are derived from a human sequence.

THSD7A-binding fragments of an anti-THSD7A antibody may also be used in the therapeutic methods of the invention as long as these fragments specifically bind to THSD7A and have the capability to block the function of THSD7A on the tumor cells. An antigen-binding fragment of an anti-THSD7A antibody may be a Fv, Fab, F(ab') and F(ab')2 fragment. Further included are single chain Fv antibody fragments and disulfide-linked Fv fragments (dsFv). Single chain antibody fragments (scFv) comprise the variable domain of the heavy chain (VH) and variable region of the light chain (VL) of an antibody, wherein these regions are linked by a peptide bridge or by disulfide bond. Methods for recombinantly producing antibody fragments have been described in the prior art and comprise, for example, techniques such as phage display or ribosome display. The recombinant antibody fragments produced by these methods may be purified and tested for binding affinity to THSD7A.

A therapeutic anti-THSD7A antibody of the invention or a fragment thereof exhibits a binding affinity for THSD7A which is higher than that for other cell-surface proteins. Preferably, the anti-THSD7A antibody of the present invention will have a binding affinity for THSD7A which is at least 2, 5, 10, 15, 20, 25, 50, 100 or at least 1000 times higher than its binding affinity for any other cell surface receptor protein.

The compound that selectively binds to THSD7A, e.g. the anti-THSD7A antibody or nanobody, may be coupled with one or more cytotoxic agents for guiding these cytotoxic agents to the tumor to be treated. Suitable cytotoxic agents that may be used in association with the THSD7A homing molecule include, but are not limited to, alkylating agents; alkyl sulfonates; aziridines, such as Thiotepa; ethyleneimines; anti-metabolites; folic acid-analogues, such as methotrexate (Farmitrexat®, Lantarel®, METEX®, MTX Hexal®); purine analogues, such as azathioprine (Azaiprin®, AZAMEDAC®, Imurek®, Zytrim®), cladribin (Leustatin®), fludarabin phosphate (Fulda®), mercapto purine (MERCAP®, Puri-Nethol®), pentostatin (Nipent®), thioguanine (Thioguanin-Wellcome®) or fludarabine; pyrimidine analogues, such as cytarabin (Alexan®, ARA-cell®, Udicil®), fluorouracil, 5-FU (Efudix®, Fluoroblastin®, Ribofluor®), gemcitabine (Gemzar®), doxifluridine, azacitidine, carmofur, 6-azauridine, floxuridine; nitrogen-lost-derivatives, such as chlorambucil (Leukeran®), melphalan (Alkeran®), chlornaphazine, estramustin, mechlorethamine; oxazaphosphorines, such as cyclophosphamide (CYCLO-cell®, Cyclostin®, Endoxan®), ifosfamide (Holoxan®, IFO-cell®) or trofosfamide (Ixoten®); nitrosureas, such as Bendamustine (Ribomustin®), Carmustine (Carmubris®), Fotemustine (Muphoran®), Lomustine (Cecenu®, Lomeblastin®), Chlorozotocine, Ranimustine or Nimustine (ACNU®); hydroxy-ureas (Litalir®); taxens, such as docetaxel (Taxotere®), or paclitaxel (Taxol®); platinum-compounds, such as cisplatin (Platiblastin®, Platinex®) or carboplatin (Carboplat®, Ribocarbo®); sulfonic acid esters, such as busulfan (Myleran®), piposulfan or treosulfan (Ovastat®); anthracyclines, such as doxorubicin (Adriblastin®, DOXO-cell®), daunorubicin (Daunoblastin®), epirubicin (Farmorubicin®), idarubicin (Zavedos®), amsacrine (Amsidyl®) or mitoxantrone (Novantron®); as well as derivates, tautomers and pharmaceutically active salts of the aforementioned compounds.

The THSD7A-binding compound, e.g. the antibody or nanobody, can be used in a method for treating a kidney, prostate, thyroid, urinary bladder, esophagus, colon, breast, or any THSD7A-expressing cancer. Preferably, the cancer to be treated has been analyzed and was found to express THSD7A. More preferably, the THSD7A expression of the tumor cells is at least 5-fold, 10-fold, 20-fold, 50-fold, 100-fold, 500-fold, 1000-fold, or 5000-fold higher than the THSD7A expression in the corresponding tissue type of a healthy subject that is not afflicted with cancer.

The therapeutically effective amount of the THSD7A-binding compound to be administered to the subject will depend on several parameters, such as the mode of administration, the particular tumor to be treated, the severity of the disease, the history of the disease, the age, height, weight, health, and physical condition of the subject to be treated, and the like.

A therapeutically effective amount of the THSD7A-binding compound can be determined by one of ordinary skill in the art without undue experimentation given the disclosure set out herein. For instance, where an anti-THSD7A antibody is used, this antibody will preferably be administered to a subject in an amount that is sufficient to achieve a plasma concentration from about 0.05 µg/ml to about 150 µg/ml, preferably from about 0.1 to about 50 µg/ml, more preferably from about 1 µg/ml to about 20 µg/ml, and usually from about 1 µg/ml to about 10 µg/ml, e.g. 5 µg/ml. In other words, the weekly dose can vary from about 1 mg to about 500 mg antibody per m² of the patient's body surface. Preferably, the weekly dose of the anti-THSD7A antibody will be from about 10-300 mg/m², more preferably from 100-200 mg/m², most preferably 150 mg/m². Where the administered compound is a fragment of an anti-THSD7A antibody, the amount to be administered can readily be adjusted based on the mass of the fragment relative to the mass of the whole antibody. The anti-THSD7A antibody of the invention can be administered in several ways (see below). Preferably, the anti-THSD7A antibody is administered by injection or infusion.

The THSD7A-binding compound, e.g. the antibody or nanobody, will normally be administered to the subject to be treated in the form of a pharmaceutical composition that is adapted to a specific route of administration. For example, pharmaceutical compositions suitable for injection and/or infusion include solutions or dispersions and powders for the extemporaneous preparation of injectable solutions or dispersions. The composition for injection must be sterile and should be stable under the conditions of manufacturing and storage. Preferably, the compositions for injection and/or infusion also include a preservative, such as a chlorobutanol, phenol, ascorbic acid, thimerosal, and the like. For intravenous administration, suitable carriers may comprise physiological saline, bacteriostatic water, Cremophor EL™ (BASF) or phosphate buffered saline (PBS). Sterile solutions for injection and/or infusion can be prepared by incorporating the active ingredient (e.g. the antibody) in the required amount in an appropriate solvent followed by filter sterilization.

In yet another aspect, the invention provides methods that make use of detection of the expression of THSD7A for diagnostic and prognostic purposes. Hence, the invention provides a method of diagnosing cancer, comprising:
(a) determining in a tissue sample of a first individual who is suspected of having cancer the expression of a THSD7A protein, preferably a protein comprising or consisting of the sequence of SEQ ID NO:1 or a variant sequence having at least 90% sequence identity thereto or a fragment of any of these sequences;
(b) comparing said expression of said sequence with the expression of the sequence in a corresponding tissue sample of a second, healthy individual,
wherein a difference of expression between the first and second individual indicates that the first individual has cancer or has an increased risk of developing cancer, if the expression in the first individual is higher than expression in the second individual. The determination of expression can be performed in accordance with techniques that are well known in the art, e.g. by PCR, RT-PCR, immunofluorescence staining or the like.

The diagnostic method provides information whether the first individual has cancer or has an increased risk of developing cancer. The presence of a cancerous disease or an increased risk for cancer is indicated by an expression of a THSD7A protein in the first individual which is at least 5-fold, at least 10-fold, at least 50-fold, at least 100-fold, or at least 1000-fold higher than the expression in the healthy reference individual.

The above method can of course be modified by using a reference from an individual who has been diagnosed with a THSD7A-expressing cancer in the specific tissue that is used for the analysis. Therefore, in still another aspect, the invention provides a method of diagnosing cancer, comprising:
(a) determining in a tissue sample of a first individual who is suspected of having cancer the expression of a THSD7A protein, preferably a protein comprising or consisting of the sequence of SEQ ID NO:1 or a variant sequence having at least 90% sequence identity thereto or a fragment of any of these sequences;
(b) comparing said expression of said sequence with the expression of the sequence in a corresponding tissue sample of a second individual who is afflicted with cancer,
wherein essentially equal levels of expression between the first and second individual indicates that the first individual has cancer or has an increased risk of developing cancer.

In still another aspect, the invention pertains to the use of a compound that selectively binds to a THSD7A protein for the diagnosis or prognosis of cancer.

The detection of a THSD7A protein, such as the protein of SEQ ID NO:1 or a variant sequence having at least 90% sequence identity thereto or a fragment of any of these sequences, on the surface of cancer cells can also be used for differentiating between different tumor types, e.g. in differential diagnosis. For example, most of the clear cell renal carcinomas are negative for THSD7A, while most of the chromophobe renal carcinoma express THSD7A. Thus, THSD7A expression can be reliably used for distinguishing these two types of renal carcinoma from each other. Thus in another aspect, the invention refers to a method of distinguishing certain tumor types from each other, preferably to a method of distinguishing clear cell renal carcinoma from chromophobe renal carcinoma by detecting the expression of THSD7A. In yet another aspect, the invention pertains to the use of a compound that selectively binds to a THSD7A protein, preferably to the human THSD7A protein of SEQ ID NO:1 or a fragment thereof, for use in tumor differential diagnosis.

### DESCRIPTION OF THE FIGURES

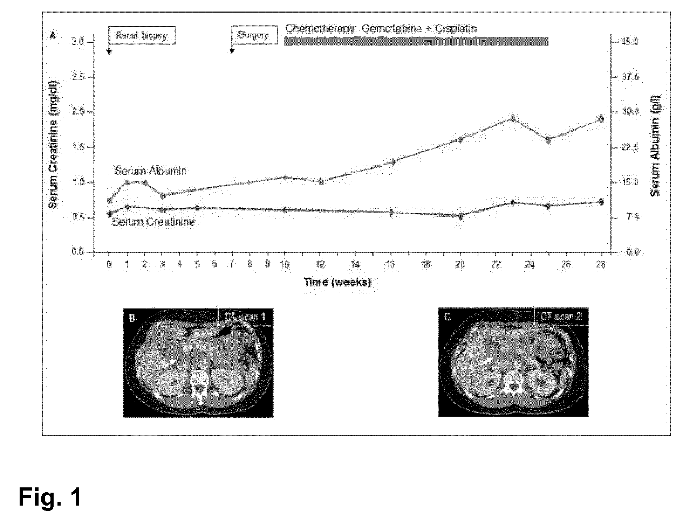

Figure 1 shows the serum creatinine and serum albumin levels and CT scans of the tumor in patient A. Serum creatinine remained within normal values during the follow-up. Serum albumin increased from 11 g/l at the time when MN was diagnosed to 29 g/l eighteen weeks after start of chemotherapy. Panel B: The CT-scan three weeks after diagnosis of MN shows a carcinoma in the gallbladder (*) and enlarged lymph nodes (arrow). Panel C: Following surgery and upon start of chemotherapy, the lymph node metastases showed no progression (arrow).

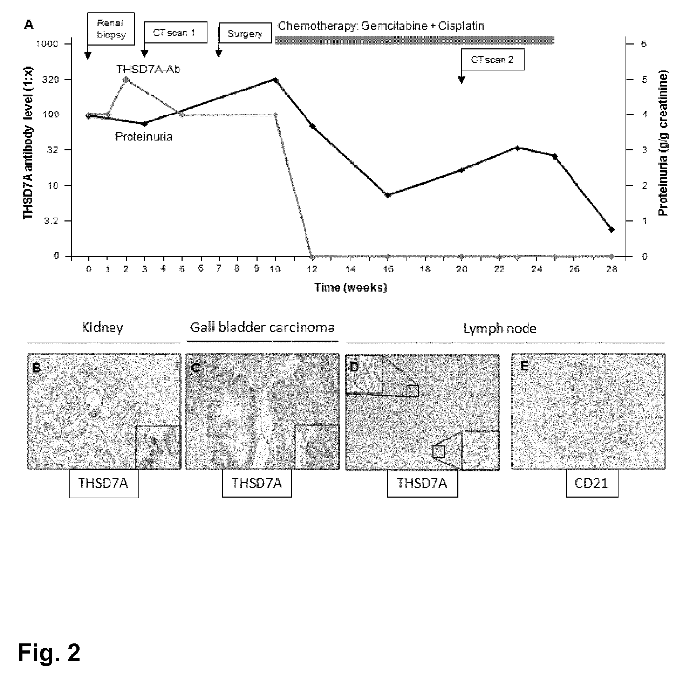

Figure 2 shows serum THSD7A antibody levels and proteinuria over time and morphologic characteristics of the patient. Panel A: THSD7A antibody levels and proteinuria were elevated at the time of diagnosis of MN. Two weeks after start of chemotherapy with Gemcitabine and Cisplatin, THSD7A antibody levels fell followed by a decrease in proteinuria. At 28 weeks THSD7A antibody levels were not detectable and proteinuria was reduced to 0.7 grams/gram creatinine. Panel B: Immunohistochemical analysis of the renal biopsy showed a strongly enhanced glomerular THSD7A staining. Panel C: Mixed adenoneuroendocrine carcinoma (MANEC) of the gallbladder. The carcinoma cells were positive for THSD7A in immunohistochemical analyses (right half) while dysplastic gall bladder cells showed a markedly lower positivity for THSD7A (left half). Panel D: Immunohistochemical analyses of lymph nodes infiltrated by metastases of the gall bladder carcinoma showed that the metastatic cells (lower right) as well as the follicular dendritic cells (upper left) stained positive for THSD7A. Panel E: Immunohistochemical analysis showing the CD21 expressing cells of the lymph node infiltrated by metastases of the gall bladder carcinoma, confirming that these are follicular dendritic cells.

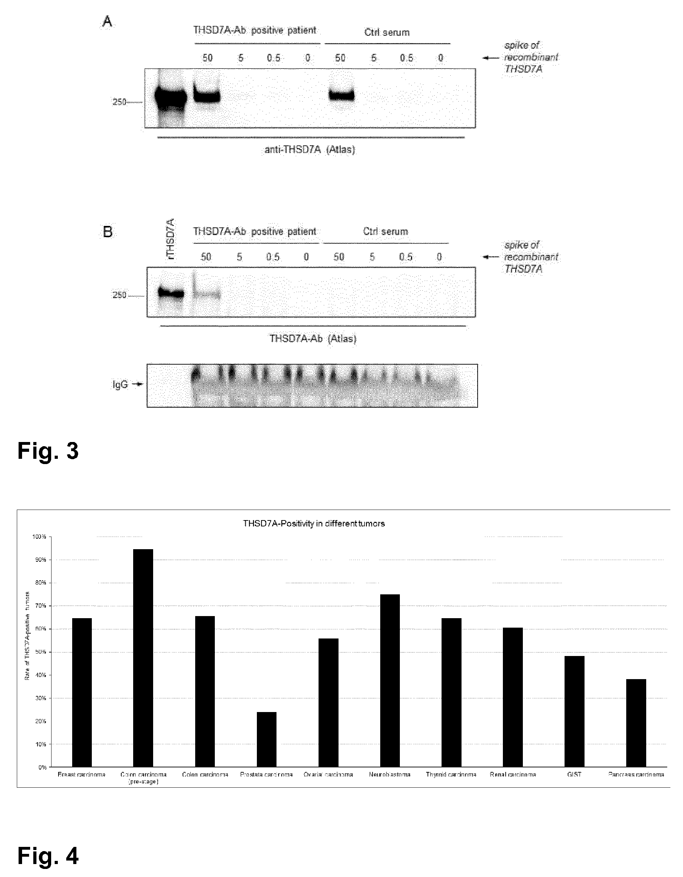

Figure 3 shows the result of the analysis of circulating THSD7A-THSD7A antibody immune complexes. Panel A: The numbering above lanes indicates the total protein amount of THSD7A-transfected HEK293 detergent-solubilized membrane extract used for each assay (50, 5 and 0.5 µg of total protein/assay) as a positive control to ensure that THSD7A could bind WGA beads and be detected by Western blot. No band was seen in the condition without spiked THSD7A while we could detect the recombinant THSD7A protein when spiking with 5 µg of total protein. Panel B: A faint band could be seen when only 5 µg of total protein from THSD7A transfected cells was added to the THSD7A antibody positive serum. However, no band was seen in serum without spiking, or when the THSD7A antibody positive serum is replaced by serum from a healthy donor. THSD7A-Ab: THSD7A antibodies; ctrl. serum: control serum. The analysis was performed as described in Tomas et al (2014), N Engl J Med, 371:2277-87.
Figure 4 shows the proportion of THSD7A-positive tumors in different malignant diseases. It can be seen that in some tumor types, the proportion of THSD7A-positive tumors is up to 70% (see, e.g. neuroblastoma).

### EXAMPLES

The following example describes the examination of a 40-year old woman, designated "Patient A" hereinafter, who was diagnosed with membranous nephropathy (MN). Upon closer examination, antibodies against THSD7A were found in the blood of the patient. A gallbladder tumor was discovered, and said tumor as well as corresponding lymph node metastases stained positive for THSD7A.

### Example 1: Clinical history and management of the patient

Patient A presented due to lower limbs edema and weight gain in the past two months. Her clinical history was unremarkable except for uterine myomas, diagnosed five years ago and hypothyroidism of unknown etiology, which was treated with thyroid hormones. An echocardiogram showed no abnormalities and the possibility of deep venous thrombosis was ruled out by sonography. Serum creatinine and urea levels were normal. Urinalysis showed proteinuria of 4.0 g/g creatinine with no hemoglobulinuria (see Figure 1A). Serum levels of C3, C4, carcinoembryonic antigen and cancer antigen 19-9 showed no abnormalities. Double-stranded-DNA, anti-nuclear antibodies and serologies for hepatitis A, B and C were negative. A gynecological examination revealed no abnormalities except for the myomas. Serum albumin was decreased to 11 g/l, while serum cholesterol and triglycerides were elevated to 294 mg/dl and 194 mg/dl, respectively.

### Example 2: Diagnosis of THSD7A-associated MN

MN was diagnosed in the patient by renal biopsy. A thickening of the glomerular capillary walls, a granular positivity for IgG in the immunohistochemistry and subepithelial electron dense deposits determined the diagnosis of membranous nephropathy.

### Example 3: Screening for THSD7A-antibodies in the blood

The blood of Patient A was screened for antibodies against THSD7A and PLA₂R1 by Western-Blot and immunofluorescence analysis as described in Tomas et al (2014), N Engl J Med, 371:2277-87.

Result: It was found that anti-THSD7A antibodies occurred in the blood of the patient. In contrast, no PLA₂R1-Ab could be detected in the plasma by indirect immunofluorescence, ELISA or Western blot.

### Example 4: Assay for the detection of circulating THSD7A immune complexes

In order to detect a potential circulating soluble form of THSD7A as well as THSD7A-THSD7A antibody immune complexes in the serum of the patient two different assays were performed as described in Tomas et al (2014), N Engl J Med, 371:2277-87. Briefly, for the detection of a circulating soluble form of THSD7A, 50 µl of THSD7A antibody positive patient or normal control serum were diluted 1:5 with 20 mM Tris pH 7.4, 140 mM NaCl, 1 mM CaCl₂ and mixed with wheat germ agglutinin (WGA) beads (Vector). As a positive control to ensure that THSD7A could bind WGA beads in the presence of other serum glycoproteins and can further be detected by Western blot, we used a detergent-solubilized fraction of recombinant THSD7A protein obtained from transfected HEK293 cells. THSD7A was spiked in the different sera in different amounts (50, 5 and 0.5 µg of HEK293 total membrane proteins) to determine the sensitivity of the assay. Samples were incubated overnight at 4°C. WGA beads were then collected by centrifugation, vigorously washed four times with 400 µl of 20 mM Tris pH 7.4, 140 mM NaCl, 1 mM CaCl₂, mixed with reducing Laemmli buffer, and boiled for 10 minutes. Samples were centrifuged and the supernatant was loaded on SDS-PAGE gels, electrophoresed, transferred to PVDF membranes, and detected with THSD7A antibody (Atlas) using high sensitivity ECL detection (Thermo Scientific).

In the second assay to detect circulating immune complexes consisting of soluble THSD7A and THSD7A antibodies, 50 µl of serum from the THSD7A antibody positive patient or serum from a healthy donor were diluted 1:5 with 20 mM Tris pH 7.4, 140 mM NaCl, 1 mM CaCl₂ and mixed with 30 µl of Protein G Sepharose (GenScript). As a positive control to ensure that the assay is able to detect THSD7A-THSD7A antibody immune complexes, sera were spiked with small amounts of solubilized recombinant THSD7A as described above. Samples were incubated overnight at 4°C. Protein G beads were then collected by centrifugation, vigorously washed four times with 400 µl of 20 mM Tris pH 7.4, 140 mM NaCl, 1 mM CaCl₂, mixed with reducing Laemmli buffer, and boiled for 10 minutes. Samples were centrifuged and the supernatant was loaded on SDS-PAGE gels, electrophoresed, transferred to PVDF membranes, and detected with THSD7A antibody (Atlas) using high sensitivity ECL detection (Thermo Scientific). Membranes were then reprobed with anti-human IgG antibodies to demonstrate efficient pull-down of serum antibodies for all samples.

Results: No circulating THSD7A-THSD7A-Ab immune complexes were detectable in the serum (see Figure 3)

### Example 5: Immunohistochemical analysis of renal biopsy

To determine whether THSD7A is the target antigen and the antibodies detected in the blood might be causative for the MN, a renal tissue biopsy was examined by THSD7A and PLA₂R1 staining as follows. Slides with kidney tissue were deparaffinized, pre-treated in citrate buffer (pH 6.1) for 15 min at 120°C and cooled down in iced water (10 min). After rinsing in 100% ethanol, slides were incubated 10 min with normal serum (Vector S2000) followed by THSD7A antibody (Sigma, rabbit, 1:4000 in antibody diluent, Invitrogen) or PLA₂R1 antibody (Sigma, 1:4000) over night at 4°C. The slides were then washed in PBS and incubated with polymer 1 (Zytomed Zytochem-Plus AP Polymer-Kit POLAP), rinsed in PBS and incubated with polymer 2 (Zytomed Zytochem-Plus AP Polymer-Kit POLAP). After washing in PBS, slides were stained in new fuchsin naphthol As-Bi phosphate substrate mixture (10 min) followed by 1 min nuclear staining in hemalaun (Mayer).

Result: The analysis revealed an increased immunohistological staining of THSD7A in the kidney of the patient (see Figure 2B). Based on the findings of Examples 1-5, it was concluded that the patient suffers from a THSD7A-associated form of MN.

### Example 6: Diagnosis of a gallbladder tumor

Three weeks after renal biopsy and diagnosis of MN, the patient presented again because of discomfort in the upper right abdomen. A computed tomogram (CT) scan showed a tumor in the gallbladder and enlarged lymph nodes around the portal vein (see Figure 1B). The gallbladder and three lymph nodes from the upper margin of the pancreas were surgically removed. Histopathologic examination revealed a mixed adenoneuroendo-crine carcinoma (MANEC) of the gallbladder with three corresponding lymph node metastases. The infiltrated lymph nodes were non-regional and therefore considered as distant metastases. The tumor classification was: G3, pT2, M1 (LYM), L1, V0, Pn0 - tumor-free resection margin.

### Example 7: Immunohistochemical analysis of tumor tissue

Tissue samples of the removed gallbladder and lymph nodes were analyzed by staining for THSD7A essentially as outlined in Example 5 above, with the exception that the THSD7A antibody was diluted 1:500 in antibody diluent rather than 1:4000.

Results: The primary gallbladder tumor cells were positive for THSD7A in the immunohistochemical analysis (see Figure 2C). Likewise, the metastatic cells as well as follicular dendritic cells stained positive for THSD7A (see Figure 2D). Follicular dendritic cells are unique stromal cells that present stored antigens to maturing B cells for high affinity antibody formation. The nature of these cells was confirmed by detection of CD21 expression (see Figure 2E).

### Example 8: RT-PCR analysis of the tumor tissue

Tissue samples of the removed gallbladder and lymph nodes were analyzed by RT-PCR analysis. RNA isolation was performed based on 10 µM paraffin-embedded tissue sections with the "High Pure FFPE RNA Microkit" (Roche, Mannheim, Germany) according to the manufacturer's recommendations. 10 µl total RNA of the different samples were reverse transcribed by 200 U "Revert Aid" reverse transcriptase (Thermo Fisher Scientific, Schwerte, Germany) using 100 ng of a random hexamer primer mix (Life Technologies, Darmstadt, Germany) for one hour at 42°C. Real time PCR was performed in a "Step One-Real-Time PCR-System" (Life Technologies, Darmstadt, Germany) using 1 µl human THSD7A-specific (GenBank Accession No: MN_015204) Taqman-probes contained in the kit (Thermo Fisher Scientific, Nr.: Hs 00324295_m1), 10 µl 2 x Fast Universal PCR Mastermix (both Life Technologies) and 8 µl cDNA. For normalization of different RNA/cDNA qualities, "SYBR-Green" based real-time PCR (Eurogentec, Köln, Germany) using 1:100 diluted cDNA and human 18S rRNA-specific primers was performed.

Results: RT-PCR analysis showed that THSD7A mRNA was detectable in the gallbladder carcinoma, but not in the normal tissue of the gallbladder. Metastases and follicular dendritic cells (fDC) of metastases-infiltrated lymph nodes were also positive for THSD7A. See Figure 2D and 2E.

### Example 9: FISH analysis

For FISH analysis, a commercial kit was utilized (Histology FISH Accessory KIT, DAKO). A Spectrum Green-labelled THSD7A-probe (Empire Genomics) was used together with a Spectrum Orange-labelled CEP7-probe (Vysis-Abbott). Before hybridization, sections were deparaffinized, air-dried, dehydrated and then denatured at 72°C for five minutes in a ThermoBrite (Vysis-Abbott). After overnight hybridization at 37°C in a ThermoBrite, slides were washed and counterstained with 10 µl DAPI (Vysis-Abbott).

Results: In FISH analysis, a polysomy of chromosome 7 was recognized with six copies of the THSD7A gene and chromosome 7 centromere.

### Example 10: Chemotherapy

Patient A was subjected to 6 cycles of chemotherapy with gemcitabine and cisplatin. Briefly, the patient received 50 mg cisplatin and 2000 mg gemcitabine on day 1 and day 8 of each cycle.

Result: Two weeks after start of chemotherapy, antibodies against THSD7A were no longer detectable in the blood, and the patient remained negative throughout the follow-up period. Four weeks later proteinuria had decreased to 1.8 g/g creatinine. Eighteen weeks after commencement of chemotherapy proteinuria had decreased to 0.7 g/g creatinine while serum albumin increased to 29 g/l. See Figure 2A. At the same time, the lymph node metastases showed no progress remaining at a constant size. See Figure 1C.

### Example 11: Screening for further tumors expressing THSD7A

Over 3.000 paraffin embedded tissue samples of human cancers were analyzed for THSD7A expression as outlined above in Example 7. This analysis concluded several (over 50) malignant tumor entities as well as benign lesions. THSD7A expression analysis was performed with a commercial probe (pH: 7.8, dilution: 1:150).

Result: Positive staining was observed in a number of malignant tumors, including chromophobe and papillary renal cell carcinoma, prostate carcinoma, medullary thyroid carcinoma, esophagus carcinoma, and medullary mammary carcinoma. In addition, THSD7A expression was also found in benign tumors such as colon adenoma and renal oncocytoma.

### Example 12: Screening sera from patients with MN

Sera from 1009 additional patients with MN were analysed by indirect immunofluorescence for the presence of THSD7A-Ab. 25 patients were found to be THSD7A-Ab positive. In all these patients, the positivity for THSD7A-Ab in the blood was confirmed by western blot, as described in Tomas et al (2014), N Engl J Med, 371:2277-87.

Result: Seven of those 25 patients, including Patient A, had a malignant tumor. These findings support a causal relationship between the expression of THSD7A in a tumor and the development of membranous nephropathy, thus describing a potential mechanism for the association between malignancy and membranous nephropathy.

## Claims

1. Method for determining whether a subject is afflicted with cancer or is susceptible to developing cancer, comprising
a) providing a blood sample from a subject;
b) determining the presence of antibodies against a Thrombospondin Type-1 Domain-containing 7A (THSD7A) protein in said blood sample;
wherein the presence of antibodies against the THSD7A protein indicates that said subject is afflicted with cancer or is susceptible to developing cancer.

2. Method according to claim 1, wherein the THSD7A protein comprises the sequence of SEQ ID NO:1 or a sequence having at least 90% sequence identity thereto or a fragment of any of these sequences.

3. Method according to claim 1 or 2, wherein the subject who is suspected to suffer from cancer is afflicted with membranous nephropathy.

4. Method according to claims 1-3, wherein said cancer is selected from, but not restricted to, kidney, prostate, thyroid, bladder, esophagus, colon or breast cancer.

5. A method of screening drug candidates to identify a drug that is useful for the treatment of cancer, comprising:
a) providing a cell that expresses a Thrombospondin Type-1 Domain-containing 7A (THSD7A) protein,
b) adding a drug candidate to said cell; and
c) determining the effect of said drug candidate on the expression of said sequence or sequence fragment or on growth, survival, or motility of the cell;
wherein the reduction of expression or reduction of growth, survival, or motility of the cell indicates that the drug candidate may be useful as a drug for the treatment of cancer.

6. Method according to claim 5, wherein the Thrombospondin Type-1 Domain-containing 7A (THSD7A) protein comprises or consists of the sequence of SEQ ID NO:1 or a sequence having at least 90% sequence identity thereto or a fragment of any of these sequences.

7. Compound that selectively binds to a Thrombospondin Type-1 Domain-containing 7A (THSD7A) protein for use in a method of treating a THSD7A-expressing cancer disease.

8. Compound that selectively binds to a Thrombospondin Type-1 Domain-containing 7A (THSD7A) protein for use in a method of claim 7, wherein said compound is coupled with a cytotoxic agent.

9. Compound that selectively binds to a Thrombospondin Type-1 Domain-containing 7A (THSD7A) protein for use in a method of claim 7 or 8, wherein said compound is an antibody, an antibody fragment, a nanobody or a nanobody fragment.

10. Compound that selectively binds to a Thrombospondin Type-1 Domain-containing 7A (THSD7A) protein for use in a method of claim 7 or 8, wherein cancer is selected from kidney, prostate, thyroid, bladder, esophagus, colon or breast cancer.

11. Pharmaceutical composition comprising a compound that selectively binds to a Thrombospondin Type-1 Domain-containing 7A (THSD7A) protein for use in a method of treating a THSD7A-expressing cancer disease.

12. Method of diagnosing cancer, comprising:
(a) determining in a tissue sample of a first individual who is suspected of having cancer the expression of a Thrombospondin Type-1 Domain-containing 7A (THSD7A) protein, preferably a protein comprising or consisting of the sequence of SEQ ID NO:1 or a variant sequence having at least 90% sequence identity thereto or a fragment of any of these sequences;
(b) comparing said expression of said sequence with the expression of the sequence in a corresponding tissue sample of a second, healthy individual,
wherein a difference of expression between the first and second individual indicates that the first individual has cancer or has an increased risk of developing cancer, if the expression in the first individual is higher than expression in the second individual.

13. Method of claim 12, wherein expression of the sequence of SEQ ID NO:1 or a variant sequence having at least 90% sequence identity thereto or a fragment of any of these sequences is determined by PCR, RT-PCR, immunofluorescence staining, immunohistochemistry, FISH.

14. Method of claim 12 or 13, wherein the expression in the first individual is at least 5-fold, at least 10-fold, at least 50-fold, at least 100-fold, at least 1000-fold higher than the expression in the healthy individual.

15. Use of a compound that selectively binds to a Thrombospondin Type-1 Domain-containing 7A (THSD7A) protein, preferably a protein comprising the sequence of SEQ ID NO:1 or a variant sequence having at least 90% sequence identity thereto or a fragment of any of these sequences, for the in vitro diagnosis of cancer.


SEQUENCE LISTING

<110>  Universitätsklinikum Hamburg-Eppendorf

<120>  NEW TARGET FOR CANCER THERAPY

<130>  P 99763

<160>  2

<170>  PatentIn version 3.5

<210>  1
<211>  1657
<212>  PRT
<213>  Homo sapiens

<400>  1

Met Gly Leu Gln Ala Arg Arg Trp Ala Ser Gly Ser Arg Gly Ala Ala
1               5                   10                  15

Gly Pro Arg Arg Gly Val Leu Gln Leu Leu Pro Leu Pro Leu Pro Leu
            20                  25                  30

Pro Leu Leu Leu Leu Leu Leu Leu Arg Pro Gly Ala Gly Arg Ala Ala
        35                  40                  45

Ala Gln Gly Glu Ala Glu Ala Pro Thr Leu Tyr Leu Trp Lys Thr Gly
    50                  55                  60

Pro Trp Gly Arg Cys Met Gly Asp Glu Cys Gly Pro Gly Gly Ile Gln
65                  70                  75                  80

Thr Arg Ala Val Trp Cys Ala His Val Glu Gly Trp Thr Thr Leu His
                85                  90                  95

Thr Asn Cys Lys Gln Ala Glu Arg Pro Asn Asn Gln Gln Asn Cys Phe
            100                 105                 110

Lys Val Cys Asp Trp His Lys Glu Leu Tyr Asp Trp Arg Leu Gly Pro
        115                 120                 125

Trp Asn Gln Cys Gln Pro Val Ile Ser Lys Ser Leu Glu Lys Pro Leu
    130                 135                 140

Glu Cys Ile Lys Gly Glu Glu Gly Ile Gln Val Arg Glu Ile Ala Cys
145                 150                 155                 160

Ile Gln Lys Asp Lys Asp Ile Pro Ala Glu Asp Ile Ile Cys Glu Tyr
                165                 170                 175

Phe Glu Pro Lys Pro Leu Leu Glu Gln Ala Cys Leu Ile Pro Cys Gln
            180                 185                 190

Gln Asp Cys Ile Val Ser Glu Phe Ser Ala Trp Ser Glu Cys Ser Lys
        195                 200                 205

Thr Cys Gly Ser Gly Leu Gln His Arg Thr Arg His Val Val Ala Pro
    210                 215                 220

Pro Gln Phe Gly Gly Ser Gly Cys Pro Asn Leu Thr Glu Phe Gln Val
225                 230                 235                 240

Cys Gln Ser Ser Pro Cys Glu Ala Glu Glu Leu Arg Tyr Ser Leu His
                245                 250                 255

Val Gly Pro Trp Ser Thr Cys Ser Met Pro His Ser Arg Gln Val Arg
            260                 265                 270

Gln Ala Arg Arg Arg Gly Lys Asn Lys Glu Arg Glu Lys Asp Arg Ser
        275                 280                 285

Lys Gly Val Lys Asp Pro Glu Ala Arg Glu Leu Ile Lys Lys Lys Arg
    290                 295                 300

Asn Arg Asn Arg Gln Asn Arg Gln Glu Asn Lys Tyr Trp Asp Ile Gln
305                 310                 315                 320

Ile Gly Tyr Gln Thr Arg Glu Val Met Cys Ile Asn Lys Thr Gly Lys
                325                 330                 335

Ala Ala Asp Leu Ser Phe Cys Gln Gln Glu Lys Leu Pro Met Thr Phe
            340                 345                 350

Gln Ser Cys Val Ile Thr Lys Glu Cys Gln Val Ser Glu Trp Ser Glu
        355                 360                 365

Trp Ser Pro Cys Ser Lys Thr Cys His Asp Met Val Ser Pro Ala Gly
    370                 375                 380

Thr Arg Val Arg Thr Arg Thr Ile Arg Gln Phe Pro Ile Gly Ser Glu
385                 390                 395                 400

Lys Glu Cys Pro Glu Phe Glu Glu Lys Glu Pro Cys Leu Ser Gln Gly
                405                 410                 415

Asp Gly Val Val Pro Cys Ala Thr Tyr Gly Trp Arg Thr Thr Glu Trp
            420                 425                 430

Thr Glu Cys Arg Val Asp Pro Leu Leu Ser Gln Gln Asp Lys Arg Arg
        435                 440                 445

Gly Asn Gln Thr Ala Leu Cys Gly Gly Gly Ile Gln Thr Arg Glu Val
    450                 455                 460

Tyr Cys Val Gln Ala Asn Glu Asn Leu Leu Ser Gln Leu Ser Thr His
465                 470                 475                 480

Lys Asn Lys Glu Ala Ser Lys Pro Met Asp Leu Lys Leu Cys Thr Gly
                485                 490                 495

Pro Ile Pro Asn Thr Thr Gln Leu Cys His Ile Pro Cys Pro Thr Glu
            500                 505                 510

Cys Glu Val Ser Pro Trp Ser Ala Trp Gly Pro Cys Thr Tyr Glu Asn
        515                 520                 525

Cys Asn Asp Gln Gln Gly Lys Lys Gly Phe Lys Leu Arg Lys Arg Arg
    530                 535                 540

Ile Thr Asn Glu Pro Thr Gly Gly Ser Gly Val Thr Gly Asn Cys Pro
545                 550                 555                 560

His Leu Leu Glu Ala Ile Pro Cys Glu Glu Pro Ala Cys Tyr Asp Trp
                565                 570                 575

Lys Ala Val Arg Leu Gly Asn Cys Glu Pro Asp Asn Gly Lys Glu Cys
            580                 585                 590

Gly Pro Gly Thr Gln Val Gln Glu Val Val Cys Ile Asn Ser Asp Gly
        595                 600                 605

Glu Glu Val Asp Arg Gln Leu Cys Arg Asp Ala Ile Phe Pro Ile Pro
    610                 615                 620

Val Ala Cys Asp Ala Pro Cys Pro Lys Asp Cys Val Leu Ser Thr Trp
625                 630                 635                 640

Ser Thr Trp Ser Ser Cys Ser His Thr Cys Ser Gly Lys Thr Thr Glu
                645                 650                 655

Gly Lys Gln Ile Arg Ala Arg Ser Ile Leu Ala Tyr Ala Gly Glu Glu
            660                 665                 670

Gly Gly Ile Arg Cys Pro Asn Ser Ser Ala Leu Gln Glu Val Arg Ser
        675                 680                 685

Cys Asn Glu His Pro Cys Thr Val Tyr His Trp Gln Thr Gly Pro Trp
    690                 695                 700

Gly Gln Cys Ile Glu Asp Thr Ser Val Ser Ser Phe Asn Thr Thr Thr
705                 710                 715                 720

Thr Trp Asn Gly Glu Ala Ser Cys Ser Val Gly Met Gln Thr Arg Lys
                725                 730                 735

Val Ile Cys Val Arg Val Asn Val Gly Gln Val Gly Pro Lys Lys Cys
            740                 745                 750

Pro Glu Ser Leu Arg Pro Glu Thr Val Arg Pro Cys Leu Leu Pro Cys
        755                 760                 765

Lys Lys Asp Cys Ile Val Thr Pro Tyr Ser Asp Trp Thr Ser Cys Pro
    770                 775                 780

Ser Ser Cys Lys Glu Gly Asp Ser Ser Ile Arg Lys Gln Ser Arg His
785                 790                 795                 800

Arg Val Ile Ile Gln Leu Pro Ala Asn Gly Gly Arg Asp Cys Thr Asp
                805                 810                 815

Pro Leu Tyr Glu Glu Lys Ala Cys Glu Ala Pro Gln Ala Cys Gln Ser
            820                 825                 830

Tyr Arg Trp Lys Thr His Lys Trp Arg Arg Cys Gln Leu Val Pro Trp
        835                 840                 845

Ser Val Gln Gln Asp Ser Pro Gly Ala Gln Glu Gly Cys Gly Pro Gly
    850                 855                 860

Arg Gln Ala Arg Ala Ile Thr Cys Arg Lys Gln Asp Gly Gly Gln Ala
865                 870                 875                 880

Gly Ile His Glu Cys Leu Gln Tyr Ala Gly Pro Val Pro Ala Leu Thr
                885                 890                 895

Gln Ala Cys Gln Ile Pro Cys Gln Asp Asp Cys Gln Leu Thr Ser Trp
            900                 905                 910

Ser Lys Phe Ser Ser Cys Asn Gly Asp Cys Gly Ala Val Arg Thr Arg
        915                 920                 925

Lys Arg Thr Leu Val Gly Lys Ser Lys Lys Lys Glu Lys Cys Lys Asn

930                 935                 940

Ser His Leu Tyr Pro Leu Ile Glu Thr Gln Tyr Cys Pro Cys Asp Lys
945                 950                 955                 960

Tyr Asn Ala Gln Pro Val Gly Asn Trp Ser Asp Cys Ile Leu Pro Glu
                965                 970                 975

Gly Lys Val Glu Val Leu Leu Gly Met Lys Val Gln Gly Asp Ile Lys
            980                 985                 990

Glu Cys Gly Gln Gly Tyr Arg Tyr  Gln Ala Met Ala Cys  Tyr Asp Gln
        995                 1000                1005

Asn Gly  Arg Leu Val Glu Thr  Ser Arg Cys Asn Ser  His Gly Tyr
    1010                1015                1020

Ile Glu  Glu Ala Cys Ile Ile  Pro Cys Pro Ser Asp  Cys Lys Leu
    1025                1030                1035

Ser Glu  Trp Ser Asn Trp Ser  Arg Cys Ser Lys Ser  Cys Gly Ser
    1040                1045                1050

Gly Val  Lys Val Arg Ser Lys  Trp Leu Arg Glu Lys  Pro Tyr Asn
    1055                1060                1065

Gly Gly  Arg Pro Cys Pro Lys  Leu Asp His Val Asn  Gln Ala Gln
    1070                1075                1080

Val Tyr  Glu Val Val Pro Cys  His Ser Asp Cys Asn  Gln Tyr Leu
    1085                1090                1095

Trp Val  Thr Glu Pro Trp Ser  Ile Cys Lys Val Thr  Phe Val Asn
    1100                1105                1110

Met Arg  Glu Asn Cys Gly Glu  Gly Val Gln Thr Arg  Lys Val Arg
    1115                1120                1125

Cys Met  Gln Asn Thr Ala Asp  Gly Pro Ser Glu His  Val Glu Asp
    1130                1135                1140

Tyr Leu  Cys Asp Pro Glu Glu  Met Pro Leu Gly Ser  Arg Val Cys
    1145                1150                1155

Lys Leu  Pro Cys Pro Glu Asp  Cys Val Ile Ser Glu  Trp Gly Pro
    1160                1165                1170

Trp Thr Gln Cys Val Leu Pro Cys Asn Gln Ser Ser Phe Arg Gln
    1175                1180                1185

Arg Ser Ala Asp Pro Ile Arg Gln Pro Ala Asp Glu Gly Arg Ser
    1190                1195                1200

Cys Pro Asn Ala Val Glu Lys Glu Pro Cys Asn Leu Asn Lys Asn
    1205                1210                1215

Cys Tyr His Tyr Asp Tyr Asn Val Thr Asp Trp Ser Thr Cys Gln
    1220                1225                1230

Leu Ser Glu Lys Ala Val Cys Gly Asn Gly Ile Lys Thr Arg Met
    1235                1240                1245

Leu Asp Cys Val Arg Ser Asp Gly Lys Ser Val Asp Leu Lys Tyr
    1250                1255                1260

Cys Glu Ala Leu Gly Leu Glu Lys Asn Trp Gln Met Asn Thr Ser
    1265                1270                1275

Cys Met Val Glu Cys Pro Val Asn Cys Gln Leu Ser Asp Trp Ser
    1280                1285                1290

Pro Trp Ser Glu Cys Ser Gln Thr Cys Gly Leu Thr Gly Lys Met
    1295                1300                1305

Ile Arg Arg Arg Thr Val Thr Gln Pro Phe Gln Gly Asp Gly Arg
    1310                1315                1320

Pro Cys Pro Ser Leu Met Asp Gln Ser Lys Pro Cys Pro Val Lys
    1325                1330                1335

Pro Cys Tyr Arg Trp Gln Tyr Gly Gln Trp Ser Pro Cys Gln Val
    1340                1345                1350

Gln Glu Ala Gln Cys Gly Glu Gly Thr Arg Thr Arg Asn Ile Ser
    1355                1360                1365

Cys Val Val Ser Asp Gly Ser Ala Asp Asp Phe Ser Lys Val Val
    1370                1375                1380

Asp Glu Glu Phe Cys Ala Asp Ile Glu Leu Ile Ile Asp Gly Asn
    1385                1390                1395

Lys Asn Met Val Leu Glu Glu Ser Cys Ser Gln Pro Cys Pro Gly
    1400                1405                1410

Asp Cys Tyr Leu Lys Asp Trp Ser Ser Trp Ser Leu Cys Gln Leu
    1415                1420                1425

Thr Cys Val Asn Gly Glu Asp Leu Gly Phe Gly Gly Ile Gln Val
    1430                1435                1440

Arg Ser Arg Pro Val Ile Ile Gln Glu Leu Glu Asn Gln His Leu
    1445                1450                1455

Cys Pro Glu Gln Met Leu Glu Thr Lys Ser Cys Tyr Asp Gly Gln
    1460                1465                1470

Cys Tyr Glu Tyr Lys Trp Met Ala Ser Ala Trp Lys Gly Ser Ser
    1475                1480                1485

Arg Thr Val Trp Cys Gln Arg Ser Asp Gly Ile Asn Val Thr Gly
    1490                1495                1500

Gly Cys Leu Val Met Ser Gln Pro Asp Ala Asp Arg Ser Cys Asn
    1505                1510                1515

Pro Pro Cys Ser Gln Pro His Ser Tyr Cys Ser Glu Thr Lys Thr
    1520                1525                1530

Cys His Cys Glu Glu Gly Tyr Thr Glu Val Met Ser Ser Asn Ser
    1535                1540                1545

Thr Leu Glu Gln Cys Thr Leu Ile Pro Val Val Val Leu Pro Thr
    1550                1555                1560

Met Glu Asp Lys Arg Gly Asp Val Lys Thr Ser Arg Ala Val His
    1565                1570                1575

Pro Thr Gln Pro Ser Ser Asn Pro Ala Gly Arg Gly Arg Thr Trp
    1580                1585                1590

Phe Leu Gln Pro Phe Gly Pro Asp Gly Arg Leu Lys Thr Trp Val
    1595                1600                1605

Tyr Gly Val Ala Ala Gly Ala Phe Val Leu Leu Ile Phe Ile Val
    1610                1615                1620

Ser Met Ile Tyr Leu Ala Cys Lys Lys Pro Lys Lys Pro Gln Arg
    1625                1630                1635

Arg Gln Asn Asn Arg Leu Lys Pro Leu Thr Leu Ala Tyr Asp Gly
    1640                1645                1650

Asp Ala Asp Met
    1655

<210> 2
<211> 10607
<212> DNA
<213> Homo sapiens

<400> 2
attctgcgag gaatggcagc ggcagcagca tccccgccag aggcggcggc ggcggcggca 60

gcggccacgg ccaccaccgc ggacgctatt gttccctggt gttagacgcg ctctccctcc 120

ttctcatctg aagcgaacaa tagcaggaaa gagctttgct ttctgtactc tttgaaaaga 180

cgttccaaga gcggagaaaa attccctgcc gagcgtgcta cggctctgga ccctggagtg 240

gctgcaggcg gcatggggct gcaagccagg cgctgggcgt ccgggagccg gggcgctgcg 300

gggccgcgcc ggggcgtcct gcagctgctg ccgctgccgc tgccgctgcc gctgctcctg 360

ctgctgctgc tacgcccggg cgccggcagg gctgcggcgc agggcgaggc ggaggcgccc 420

accctctatc tgtggaagac tggtccatgg ggccgatgta tgggagatga atgtggtccc 480

ggaggcatcc aaacgagggc tgtgtggtgt gctcatgtgg agggatggac tacactgcat 540

actaactgta agcaggccga gagacccaat aaccagcaga attgtttcaa agtttgcgat 600

tggcacaaag agttgtacga ctggagactg ggaccttgga atcagtgtca gcccgtgatt 660

tcaaaaagcc tagagaaacc tcttgagtgc attaaggggg aagaaggtat tcaggtgagg 720

gagatagcgt gcatccagaa agacaaagac attcctgcgg aggatatcat ctgtgagtac 780

tttgagccca agcctctcct ggagcaggct tgcctcattc cttgccagca agattgcatc 840

gtgtctgaat tttctgcctg gtccgaatgc tccaagacct gcggcagcgg gctccagcac 900

cggacgcgtc atgtggtggc gcccccgcag ttcggaggct ctggctgtcc aaacctgacg 960

gagttccagg tgtgccaatc cagtccatgc gaggccgagg agctcaggta cagcctgcat 1020

gtggggccct ggagcacctg ctcaatgccc cactcccgac aagtaagaca agcaaggaga 1080

cgcgggaaga ataaagaacg ggaaaaggac cgcagcaaag gagtaaagga tccagaagcc 1140

cgcgagctta ttaagaaaaa gagaaacaga aacaggcaga acagacaaga gaacaaatat 1200

tgggacatcc agattggata tcagaccaga gaggttatgt gcattaacaa gacggggaaa 1260

gctgctgatt taagcttttg ccagcaagag aagcttccaa tgaccttcca gtcctgtgtg 1320

atcaccaaag agtgccaggt ttccgagtgg tcagagtgga gcccctgctc aaaaacatgc 1380

catgacatgg tgtcccctgc aggcactcgt gtaaggacac gaaccatcag gcagtttccc 1440

attggcagtg aaaaggagtg tccagaattt gaagaaaaag aaccctgttt gtctcaagga 1500

gatggagttg tcccctgtgc cacgtatggc tggagaacta cagagtggac tgagtgccgt 1560

gtggaccctt tgctcagtca gcaggacaag aggcgcggca accagacggc cctctgtgga 1620

gggggcatcc agacccgaga ggtgtactgc gtgcaggcca acgaaaacct cctctcacaa 1680

ttaagtaccc acaagaacaa agaagcctca aagccaatgg acttaaaatt atgcactgga 1740

cctatcccta atactacaca gctgtgccac attccttgtc caactgaatg tgaagtttca 1800

ccttggtcag cttggggacc ttgtacttat gaaaactgta atgatcagca agggaaaaaa 1860

ggcttcaaac tgaggaagcg gcgcattacc aatgagccca ctggaggctc tggggtaacc 1920

ggaaactgcc ctcacttact ggaagccatt ccctgtgaag agcctgcctg ttatgactgg 1980

aaagcagtga gactgggaaa ctgcgagcca gataacggaa aggagtgtgg tccaggcacg 2040

caagttcaag aggttgtgtg catcaacagt gatggagaag aagttgacag acagctgtgc 2100

agagatgcca tcttccccat ccctgtggcc tgtgatgccc catgcccgaa agactgtgtg 2160

ctcagcacat ggtctacgtg gtcctcctgc tcacacacct gctcagggaa aacgacagaa 2220

gggaaacaga tacgagcacg atccattctg gcctatgcgg gtgaagaagg tggaattcgc 2280

tgtccaaata gcagtgcttt gcaagaagta cgaagctgta atgagcatcc ttgcacagtg 2340

taccactggc aaactggtcc ctggggccag tgcattgagg acacctcagt atcgtccttc 2400

aacacaacta cgacttggaa tggggaggcc tcctgctctg tcggcatgca gacaagaaaa 2460

gtcatctgtg tgcgagtcaa tgtgggccaa gtgggaccca aaaaatgtcc tgaaagcctt 2520

cgacctgaaa ctgtaaggcc ttgtctgctt ccttgtaaga aggactgtat tgtgacccca 2580

tatagtgact ggacatcatg cccctcttcg tgtaaagaag gggactccag tatcaggaag 2640

cagtctaggc atcgggtcat cattcagctg ccagccaacg ggggccgaga ctgcacagat 2700

cccctctatg aagagaaggc ctgtgaggca cctcaagcgt gccaaagcta caggtggaag 2760

actcacaaat ggcgcagatg ccaattagtc ccttggagcg tgcaacaaga cagccctgga 2820

gcacaggaag gctgtgggcc tgggcgacag gcaagagcca ttacttgtcg caagcaagat 2880

ggaggacagg ctggaatcca tgagtgccta cagtatgcag gccctgtgcc agcccttacc 2940

caggcctgcc agatcccctg ccaggatgac tgtcaattga ccagctggtc caagttttct 3000

tcatgcaatg gagactgtgg tgcagttagg accagaaagc gcactcttgt tggaaaaagt 3060

aaaaagaagg aaaaatgtaa aaattcccat ttgtatcccc tgattgagac tcagtattgt 3120

ccttgtgaca aatataatgc acaacctgtg gggaactggt cagactgtat tttaccagag 3180

ggaaaagtgg aagtgttgct gggaatgaaa gtacaaggag acatcaagga atgcggacaa 3240

ggatatcgtt accaagcaat ggcatgctac gatcaaaatg gcaggcttgt ggaaacatct 3300

agatgtaaca gccatggtta cattgaggag gcctgcatca tcccctgccc ctcagactgc 3360

aagctcagtg agtggtccaa ctggtcgcgc tgcagcaagt cctgtgggag tggtgtgaag 3420

gttcgttcta aatggctgcg tgaaaaacca tataatggag gaaggccttg ccccaaactg 3480

gaccatgtca accaggcaca ggtgtatgag gttgtcccat gccacagtga ctgcaaccag 3540

tacctatggg tcacagagcc ctggagcatc tgcaaggtga cctttgtgaa tatgcgggag 3600

aactgtggag agggcgtgca aacccgaaaa gtgagatgca tgcagaatac agcagatggc 3660

ccttctgaac atgtagagga ttacctctgt gacccagaag agatgcccct gggctctaga 3720

gtgtgcaaat taccatgccc tgaggactgt gtgatatctg aatggggtcc atggacccaa 3780

tgtgttttgc cttgcaatca aagcagtttc cggcaaaggt cagctgatcc catcagacaa 3840

ccagctgatg aaggaagatc ttgccctaat gctgttgaga aagaaccctg taacctgaac 3900

aaaaactgct accactatga ttataatgta acagactgga gtacatgtca gctgagtgag 3960

aaggcagttt gtggaaatgg aataaaaaca aggatgttgg attgtgttcg aagtgatggc 4020

aagtcagttg acctgaaata ttgtgaagcg cttggcttgg agaagaactg gcagatgaac 4080

acgtcctgca tggtggaatg ccctgtgaac tgtcagcttt ctgattggtc tccttggtca 4140

gaatgttctc aaacatgtgg cctcacagga aaaatgatcc gaagacgaac agtgacccag 4200

ccctttcaag gtgatggaag accatgccct tccctgatgg accagtccaa accctgccca 4260

gtgaagcctt gttatcggtg gcaatatggc cagtggtctc catgccaagt gcaggaggcc 4320

cagtgtggag aagggaccag aacaaggaac atttcttgtg tagtaagtga tgggtcagct 4380

gatgatttca gcaaagtggt ggatgaggaa ttctgtgctg acattgaact cattatagat 4440

ggtaataaaa atatggttct ggaggaatcc tgcagccagc cttgcccagg tgactgttat 4500

ttgaaggact ggtcttcctg gagcctgtgt cagctgacct gtgtgaatgg tgaggatcta 4560

ggctttggtg gaatacaggt cagatccaga ccggtgatta tacaagaact agagaatcag 4620

catctgtgcc cagagcagat gttagaaaca aaatcatgtt atgatggaca gtgctatgaa 4680

tataaatgga tggccagtgc ttggaagggc tcttcccgaa cagtgtggtg tcaaaggtca 4740

gatggtataa atgtaacagg gggctgcttg gtgatgagcc agcctgatgc cgacaggtct 4800

tgtaacccac cgtgtagtca accccactcg tactgtagcg agacaaaaac atgccattgt 4860

gaagaagggt acactgaagt catgtcttct aacagcaccc ttgagcaatg cacacttatc 4920

cccgtggtgg tattacccac catggaggac aaaagaggag atgtgaaaac cagtcgggct 4980

gtacatccaa cccaaccctc cagtaaccca gcaggacggg gaaggacctg gtttctacag 5040

ccatttgggc cagatgggag actaaagacc tgggtttacg gtgtagcagc tggggcattt 5100

gtgttactca tctttattgt ctccatgatt tatctagctt gcaaaaagcc aaagaaaccc 5160

caaagaaggc aaaacaaccg actgaaacct ttaaccttag cctatgatgg agatgccgac 5220

atgtaacata taacttttcc tggcaacaac cagtttcggc tttctgactt catagatgtc 5280

cagaggccac aacaaatgta tccaaactgt gtggattaaa atatatttta atttttaaaa 5340

atggcatcat aaagacaaga gtgaaaatca tactgccact ggagatattt aagacagtac 5400

cacttatata cagaccatca accgtgagaa ttataggaga tttagctgaa tacatgctgc 5460

attctgaaag ttttatgtca tcttttctga aatctaccga ctgaaaaacc actttcatct 5520

ctaaaaaata atggtggaat tggccagtta ggatgcctga tacaagaccg tctgcagtgt 5580

taatccataa aacttcctag catgaagagt ttctaccaag atctccacaa tactatggtc 5640

aaattaacat gtgtactcag ttgaatgaca cacattatgt cagattatgt acttgctaat 5700

aagcaatttt aacaatgcat aacaaataaa ctctaagcta agcagaaaat ccactgaata 5760

aattcagcat cttggtggtc gatggtagat tttattgacc tgcatttcag agacaaagcc 5820

tcttttttaa gacttcttgt ctctctccaa agtaagaatg ctggacaagt actagtgtct 5880

tagaagaacg agtcctcaag ttcagtattt tatagtggta attgtctgga aaactaattt 5940

acttgtgtta atacaatacg tttctacttt ccctgatttt caaactggtt gcctgcatct 6000

tttttgctat atggaaggca catttttgca ctatattagt gcagcacgat aggcgcttaa 6060

ccagtattgc catagaaact gcctcttttc atgtgggatg aagacatctg tgccaagagt 6120

ggcatgaaga catttgcaag ttcttgtatc ctgaagagag taaagttcag tttggatggc 6180

agcaagatga aatcagctat tacacctgct gtacacacac ttcctcatca ctgcagccat 6240

tgtgaaattg acaacatggc ggtaatttaa gtgttgaagt ccctaacccc ttaaccctct 6300

aaaaggtgga ttcctctagt tggtttgtaa ttgttctttg aaggctgttt atgactagat 6360

ttttatattt gttatctttg ttaagaaaaa aaaagaaaaa ggaactggat gtctttttaa 6420

ttttgagcag atggagaaaa taaataatgt atcaatgacc tttgtaacta aaggaaatca 6480

aaattatatg ttgatttttc tttctctctg atttcccagt ttcagattga atgtctgtct 6540

tgcaggcagt tatttcaaaa tccatagtct tttgcctttc tcactggcaa aatttgaagc 6600

aatctctctc ttcaatgtat gatttcaaaa ctaaaatttt aaaagagaag aataaatatc 6660

ttatccaaga cttcattgca tatctagagc aagagtggca aactttttct gtaaaggact 6720

atatagaaaa tattttccac aaggtctatg ttgcaacttc tcaactttgc cattctgcta 6780

atgcaatctt agtaaataaa agaatgaaca taactgtttt cctatataac ttcccttaca 6840

aaaacagcca gtaggccacc tttgcaggtc atattttgct gacccctggt ctggatgttt 6900

tttaatgcta gctctccatg gtgtcatctt tcgttttcat tcatttttca aaggtgagaa 6960

tttacagtca ttacatttcc cactgtatca gaagtagctg tattttctac agatgtagcc 7020

ctgggaatgg cacagtggaa caatatttaa ctaactcagc agacttcttg caaatgaaaa 7080

gaaaactcaa aaggtgccat taaaaagcaa taccattttc aggcaacatt attgcaacaa 7140

cctttgtgag agaggacact agcttttcag gatatttgaa aaataatata actaacatgc 7200

tattttatct taaacagctg tgttcctgta aaagtgtttt ggattagaag gtatttttac 7260

cttcatactg gagtatgaac tccccctgct gttcattaga gtatttgcaa agattacaaa 7320

agacatcagg ttaggatgtc attgtcccca aaatgaaaat atttgtatgt atttcaataa 7380

gaactaaatg cagtaagcgc taattcctca ttcaatattc actatattag gaaatgtaaa 7440

tcatttcaaa gagaacaggt agtcaggaag atgaaaattt tactttaaag caaggattta 7500

aacaaagaaa accataaaaa caaagtccat tttatctgtt ttattaaaga ggaagaaaaa 7560

ataatattgt tactttaaaa ctgtgattgt caatatatat aatcaggggt cttccagata 7620

atcctaaagt tgatctcttt gaataaaata aactccattt gtccaaaata gttaagaaag 7680

actctattgt ggttacttga aaaattcagc taaccctctt atgaaatgtg tgcaacacct 7740

attaattttg aaatgtggga tagaatacct actaaagcta tatttaaggt attgtaaaat 7800

gtcattaatt cagaaaagat tagtatcatc ctacctataa gaattaaatt agcaaatcaa 7860

aattatagaa tgttatgaaa aatacatttt attcataaat taattagatt ttacagcata 7920

tatgcatgca tatatatata tacacacaca cacacacaca cccatatata tgagagagag 7980

gaaattaggt tcaacatgac ctaatattag agaacatatt ggaaggtcac tgtttgttag 8040

gataaataaa tactggataa cctcgttaga aaggtggaaa gaggagaacg tagcaatctg 8100

gctattttgg acaaagggaa ggttattata ttcatgttta taacatgaaa atggccttat 8160

ttttgtttct gacatgagac aataatgagt taaatatttt attggaattt catcaaaacc 8220

ttatttttac tgttccttct aaaaatttgg gaggggaata tagtagtaac ttctctattg 8280

acacttttac ctcaccgagg gggtaaaaga tgtagaactg ctgttcccta gaatgaaggt 8340

ctgttgtttg gtttttaaaa aaagaagagc agaaaaagtt tgacacagta ttatatgatt 8400

ttgctggact atttcactag aaaccacgta gaataggact aactgatctc ttttgaaagg 8460

ggctgatttg cttattcatc aataactaat ccaatatgtg gacagtaatc ttaatttcaa 8520

agcctttttt gaggatacta cggtgtagct taagtgaatt taaaaagagt gcataaaagc 8580

ttgtttttaa ttacttataa caaagcacaa attacttcta acaaggcaaa tatttattga 8640

catataagac attctgctag gcattgtaaa aaaaaaaaaa aaagtaatgg cttttgtata 8700

cataggtact taagacagac atccaaaaga aggaaaaaat gcttaatgag tgaagatagc 8760

actgaaacat gttgactttt actctaggtt tgtttttaga tggggttagt attttcccct 8820

acgtaatgta catgtcttta ggccacagta ttaggagttt gatttttccc tgccccaccc 8880

tcaggtaggt ggcagtggtc attgtagctt aatcagaccc ctgttaagtt cctgggcatt 8940

tcaaggtaaa ttcacgtctt ccttctcaca gaaattgaat agacctctca cacacttatt 9000

tacctccccc aacaacctgt atccttggcc ctttctgatt ttctctgctt ggtctctttc 9060

tactcttgac agctattctt acctacttcc cactaaacat gcccaatttt ttaattaaaa 9120

aaaaaagaat tcctatattt ccttccctat tagaatcaaa gtgtcactca ctcagagtgt 9180

gaacatcctg atttacacca aatccactgg tcaaagaaat gaataaaggg aaaaggaaaa 9240

atgtggaagg catgcaggaa aaaaaaaaaa aaaaaagccc ttttctgtaa aaatgtccat 9300

gcctacactt gctttagacc atcccatatc ctcctgcctc catctcgagt ctttgttcac 9360

attccacacc acaagacagt gagggaggac tgtacttttc agtaccatac agaagggaga 9420

gcctgttcta aagctgacgt gggtctcaat agtacatgag aagcacaatt cccaaatgat 9480

ctgggatctc ctgtttataa atatattacc aaattcaagt gtgggttctt cttctatttc 9540

cactgtaaga ttgagggtgt tgaaaaaaca gtagtccttg agcaaatctg tgaggaatct 9600

tgtcagaatg tgatctttcc tgggactagt ttagttgtga gaactacatc tgcttgttct 9660

gggaataagg gtctccccag gtcctgccct caggtggccc tggctgcagg acaggatttc 9720

aggtatcaga aactggtgtg tgagccaagc aaatagttat ccatttataa aaaggatggt 9780

ttgcagtgtg ttataataat tttatatcta gcagggacaa tgaaaaatgg ttatgtctac 9840

attattaaat gaatgaaact aatgctaaac agttaatttt gcatttttat ttttctgtgt 9900

ttctaccaag tcagatctta aaaatgggca gtatatgaag agctttagtt tgtgggagtc 9960

atgttttcag tgatcaaaaa tctctatatt atttagaata taattgtcag tcttgttgtg 10020

ttttgtagat ttattgtttt taagccatag tttctagatt acctactttt aaccctcaag 10080

taaacttttc cctatttttc agaaatctgt ttgtgtttat ttttagaaag taaacattta 10140

gattcctttt ctggtacttt ttttctccat ttctatcaca cagatttgaa gagatcagta 10200

tctatcattg taaaatataa ctttattaaa atttgcaatc tgtctttcta taaattatta 10260

ttccctaaat gtgtatttta tgttaattta actacctgaa tatttatttt gtttaaataa 10320

ggaggcagct gtctaagaat ttttgttaat attactttgt aaattgcgta atgtaattta 10380

ttttacatta tgtacatttc ctttttaacg cacaaaatgt ctatgtacaa ttaactacag 10440

atttgcaaat aatgtcacta agctttattc agtcaataca gatggcatgt gaagatgtaa 10500

tatgcttttt tacattttca tatgggttat ttgtaaataa ctatattgct gacaaacata 10560

aatacggaaa tcattttcat tctaaaaaaa aaaaaaaaaa aaaaaaa 10607